# EUROPEAN PATENT APPLICATION

(11) **EP 2 422 844 A1**
(43) Date of publication of application: **29.02.2012**
(21) Application number: 10173892.0
(22) Date of filing: 24.08.2010
(51) Int. Cl.: A61N 5/06

(54) **Wearable phototherapy device**

(71) Applicant: Polyphotonix Limited, Sedgefield County, Durham TS21 3FG (GB)
(72) Inventor: Veres, Janos, Pittsford, NY 14534 (US)
(74) Representative: Vinsome, Rex Martin

(57) **Abstract**

A wearable apparatus (100) for directing electromagnetic radiation onto at least one tissue area is disclosed. The apparatus comprises at least one radiation collection surface (101) for receiving incident electromagnetic radiation and at least one radiation emitting surface (102) adapted to direct electromagnetic radiation onto at least one target area of tissue to be treated. A light guide (103) is adapted to receive electromagnetic radiation received by at least one radiation collection surface and to direct at least part of the electromagnetic radiation to at least one radiation emitting surface, wherein a total surface area of the or each radiation collection surface is larger than a total surface area of the or each radiation emitting surface. Coupling means increases the proportion of electromagnetic radiation received by at least one radiation collection surface which enters the light guide.

## Description

The current invention provides improved phototheraphy devices for therapeutic or cosmetic treatments. The devices can be patches, bandages, masks or clothing that are comfortable to wear and can be easily tuned to the condition treated and the area exposed.

### Background of the invention

Phototheraphy has been used for a wide variety of therapeutic or cosmetic purposes. Conditions that can be treated with light include chronic infections (HIV, HHV-6, Hepatitis A,B,C), candidiasis, idiopatic infections, mycoplasmal infections, bacterial infections, wounds, diabetic ulcers, pre-cancer conditions such as endometriosis, cervical dysplasia, skin conditions such as psoriasis, eczema pityriasys, fungal infections, vitiligo, pain and inflammatory conditions, edema, jaundice. Light has also been effective to treat seasonal affective disorders, depression, neuropathy and circadian rhythm disorders

In photo-dynamic therapy (PDT) specific light waves and photo-sensitizing drugs are used together to treat conditions. In describing this invention we use the terms phototherapy to include the photo-dynamic therapy.

Light has also been used for dermatology and cosmetic purposes, for example to rejuvenate skin, accelerate healing post plastic surgery as well as antiwrinkle purposes.

The above uses have been described well in the literature, for example in "Phototheraphy and photochemotheraphy by W.L. Morison, 1982, Preaeger Publishers; "Dermatological phototheraphy and photodiagnostic methods", by J. Krutmann, 2008, Springer as well as in "Phototheraphy in mental health" by D.A. Krauss and J.L. Fryrear, 1983, Charles C Thomas Pub. Ltd.

Phototheraphy is conducted by exposing tissue to certain wavelengths and doses of light. The procedure can be effectively carried out in a clinical environment whereby the patient is required to stay without movement to ensure correct exposure to light. Light sources such as incandescent lamps or lasers require a complex and bulky setup, and thus are tied to a hospital or clinical environment. More recently LEDs have been used to offer more compact solutions for phototheraphy purposes. It is desirable to provide phototheraphy devices that enable some freedom of motion for the patient or most ideally phototheraphy devices that are wearable. It is desirable that devices are thin, lightweight, and do not require heavy power sources to be carried.

US 2010/0010593 (Cacciola) describes a plaster and radiation device used for therapy. The device operates using LEDs or other electrically operated light sources and therefore requires carrying an electrical power source.

US 2004/0111132 (Shenderova) describes phototherapy devices based on the use of thin film electroluminescent (TFEL) and organic light emitting diode (OLED) light sources. These devices require power source to be carried for wearability.

US2007/0233208 (Kurtz) describes a light therapy bandage using an array of light emitters. The device requires a separate power source.

US2010/0179469 (Hammond) describes a light therapy devices using OLED light source. These devices require a separate power source.

US2007/126577 (Kurtz) describes a light guide based phototherapy device. The device requires a separate light source and a power source.

US2008/0058689 (Holloway) describes a phototherapy bandage based on TFEL or OLED light source, both requiring power sources to be carried.

US5616140 (Prescott) describes a battery powered laser bandage.

US6096066 (Chen) describes a conformal patch for light therapy based on an array of LED light sources requiring a power source.

US6,231,593 (Meserol) describes a fibre optic device to treat skin tissue. The invention requires an external controller and power source.

WO02/100484 (Lenke) describes a skin adhesive dressing requiring an external electrical power source.

WO2007/125336 (Samuel) describes a therapeutic light emitting device comprising LED or other sources requiring a power supply.

US2010/0063567 (Solsberg) describes a chemiluminescent phototherapy device based on reacting oxalic ester with hydrogen peroxide and a fluorescent solution. The device does not require an electrical power source. However, due to the fluids used the device requires protective sealing if incorporated in wearable devices. The invention does not resolve the limited emissive lifetime and brightness of the chemiluminescent source

The prior art devices based on LED and other electrically operated devices are dependent on an electrical power supply, which either ties them to mains electricity supply or requires carrying batteries. A further disadvantage of LED sources that they are small and can generate substantial heat in a localized area, causing discomfort. Chemiluminescent devices proposed by US2010/0063567 can operate without batteries; however, they are not easily integrated into lightweight wearable phototheraphy devices and are potentially associated with a risk of fluids entering in contact with tissue in the event of a leak. In addition such devices have a single use lifetime.

There is a need for improved wearable phototherapy devices that are lightweight and operate independent of an electrical power supply. Preferred embodiments of the present invention seek to overcome one or more of the disadvantages of known solutions discussed above.

### Summary of the invention

According to an aspect of the present invention, there is provided a wearable apparatus for directing electromagnetic radiation onto at least one tissue area, the apparatus comprising:
at least one radiation collection surface for receiving incident electromagnetic radiation;
at least one radiation emitting surface adapted to direct electromagnetic, radiation onto at least one target area of tissue to be treated;
radiation guide means adapted to receive electromagnetic radiation received by at least one said radiation collection surface and to direct at least part of said electromagnetic radiation to at least one said radiation emitting surface, wherein a total surface area of the or each said radiation collection surface is larger than a total surface area of the or each said radiation emitting surface; and
coupling means for increasing the proportion of electromagnetic radiation received by at least one said radiation collection surface which enters said radiation guide means.

We are surrounded by abundant electromagnetic radiation such as sunlight or artificial light. The present invention is based on the surprising discovery by the inventors that a lightweight wearable phototherapy device can be built that harnesses ambient radiation without the need for an electrical power source. In describing the invention the terms "radiation" and "light" are used interchangeably, recognizing that light is an electromagnetic radiation and can be of different wavelengths.

By providing a light collection surface it is possible to capture and utilize ambient, surrounding light for the treatment of tissue, for example the treatment of wounds. The device therefore does not require a dedicated power source and can be made lightweight and comfortable to wear. The collected ambient light is guided to the light emitting surface and delivered to the tissue. For example, the device can be formed into a bandage that protects the wound, the outer surface comprising the light collection surface and the inner surface covering the wound comprising a smaller light emitting surface delivering the harvested ambient light to the wound.

The invention in turn provides the advantage of enabling the necessary therapeutic radiation doses to be generated without a power supply, increasing the mobility of the user. In addition to collecting ambient light, the device may comprise materials exhibiting delayed phosphorescence, which enables light to be accumulated or stored so that its delivery remains possible after the ambient source of radiation has been removed. Thus the device can be exposed to ambient light to collect radiation which is then released with a delay, making it possible to wear the device under clothes. By employing a large light collection surface, the amount of light harvested can be significantly increased. The light guide and light emitting means ensure that a substantial portion of the collected light is concentrated and delivered to the area to be treated. The device might comprise dyes or optical filters to control the spectral characteristics of the treatment. The use of one or more fluorescent or phosphorescent dyes, for example, makes it possible to convert light of shorter wavelength to the desired output colour. In some cases longer wavelength light can also be converted to shorter wavelength, albeit at lower efficiency.

By suitable choice of materials, the wavelength, intensity and duration of illumination can be controlled. The present phototheraphy device based on collecting ambient light has the advantage of enabling a more comfortable and/or a disposable apparatus to be provided, for example an apparatus which emits radiation over an extended period in response to initial excitation. Such a device does not require batteries and is of little maintenance. The device can be made thin, conformal and lightweight for wearability.

The apparatus may be adapted to capture ambient light, direct at least part of said light onto a target area of tissue to be treated, and to adjust spectral properties and/or intensity of at least part of said light.

This provides the advantage of improving the ease of use and reducing the cost of manufacture of the apparatus. For example, the apparatus may capture ambient sunlight and/or ambient artificial light in a room or enclosure in which the apparatus is located.

At least one said radiation emitting surface may be provided on at least one detachable member.

The radiation guide means may comprise a radiation transmitting portion including at least one first layer having a respective first refractive index and at least one second layer having a respective second refractive index, wherein said first refractive index of at least one said first layer is higher than the respective second refractive index of at least one second layer in contact with said first layer.

This provides the advantage of minimising radiation loss out of said first layer by causing total internal reflection of the radiation.

The apparatus may further comprise spectral adjustment means for adjusting spectral characteristics of electromagnetic radiation delivered to the target treatment area.

The spectral adjustment means may include at least one material having a transmissivity to radiation varying with radiation intensity.

This provides the advantage that the response of such a photochromic layer compensates decay in the emission of at least one said radiation emitting surface. This in turn makes it possible to control or limit the light intensity delivered to the target area.

The coupling means may include at least one phosphorescent or luminescent material provided at at least one said radiation collection surface and/or said radiation guide means and/or at least one said radiation emitting surface.

This provides the advantage of enabling the predominant spectral range of radiation delivered to the target area to be selected. Luminescent materials may also improve the ability to capture ambient light irrespective of the angle of incidence.

At least one said luminescent material may be concentrated in a region adjacent at least one said radiation emitting surface.

This provides the advantage of minimising the amount of luminescent material needed to be used as well as simplifying the requirements of any dichroic reflectors used as well as ensuring that as little light as possible is lost to reabsorption within the structure.

The radiation guide means may further comprise at least one reflective portion.

This provides the advantage of further minimising leakage of radiation from the radiation guide means, as a result of which the intensity of radiation directed to treatment area can be maximized.

The radiation guide means may comprise a radiation transmitting portion having tapered thickness adjacent at least one edge portion thereof. This provides the advantage of enabling radiation leakage from the radiation guide means to be minimised under certain circumstances. For example, the radiation leakage could be minimised as a result of increasing angle of reflection from a second layer having lower refractive index adjacent to a first layer having higher refractive index, thus further contributing to total internal reflection.

The radiation guide means may comprise a radiation transmitting portion, wherein the radiation transmitting portion is rounded adjacent at least one edge portion thereof. This provides the advantage of further minimising radiation leakage.

At least one said radiation emitting surface (RES) may be adapted to conform to a shape of the tissue to be treated.

At least one said radiation emitting surface (RES) may include at least one coolable material.

At least one said radiation emitting surface may be adapted to be moved between a respective first position, in which said surface is adapted to direct radiation into said radiation guide means, and a respective second position, in which said surface is adapted to be exposed to exciting radiation.

This provides the advantage of enabling a more compact apparatus to be provided.

At least one said radiation emitting surface may be adapted to be moved between respective first and second positions therefore by folding.

The apparatus may be integrated into clothing.

The apparatus may further comprise at least one photochemical and/or photo-pharmaceutical agent.

Preferred embodiments of the invention will now be described, by way of example only and not in any limitative sense, with reference to the accompanying drawings, in which:
Figure 1 illustrates a first embodiment of a phototheraphy plaster with the top view (A) and cross-section B shown;
Figure 2 is a perspective view of the first embodiment plaster applied to an arm;
Figure 3 illustrates a phototheraphy device integrated into clothing with localised radiation emitting surfaces corresponding to the areas to be treated;
Figure 4 is a perspective view of a phototheraphy plaster with radiation collection surface and radiation emitting surfaces offset;
Figure 5 is a side cross-sectional view illustrating a device of collecting and subsequently emitting ambient light according to the current invention ;
Figure 6 is a side cross-sectional view a phototheraphy device comprising a phosphorescent top layer that is capable to absorbing and re-emitting light over prolonged time;
Figure 7 is a side cross-sectional view of phototheraphy device comprising a phosphorescent layer that positioned below the light guide;
Figures 8(i) to 8(iii) show a side cross-sectional view of a foldable phototheraphy device for increased light output;
Figure 9 is a cross-sectional view of a phototheraphy device with a tapered end lightguide;
Figure 10 is a side cross-sectional view two component device comprising a separate outcoupling layer;
Figure 11 is a perspective view of a phototherapy device comprising fibre light guides each having light collection and light emitting regions;
Figure 12 illustrates fibre based light collection and an area outcoupling portion;
Figure 13 is a perspective view of a phototherapy device for treating hayfever;
Figure 14 is a perspective view of a phototherapy device for treating the palm of the hand

### Detailed description

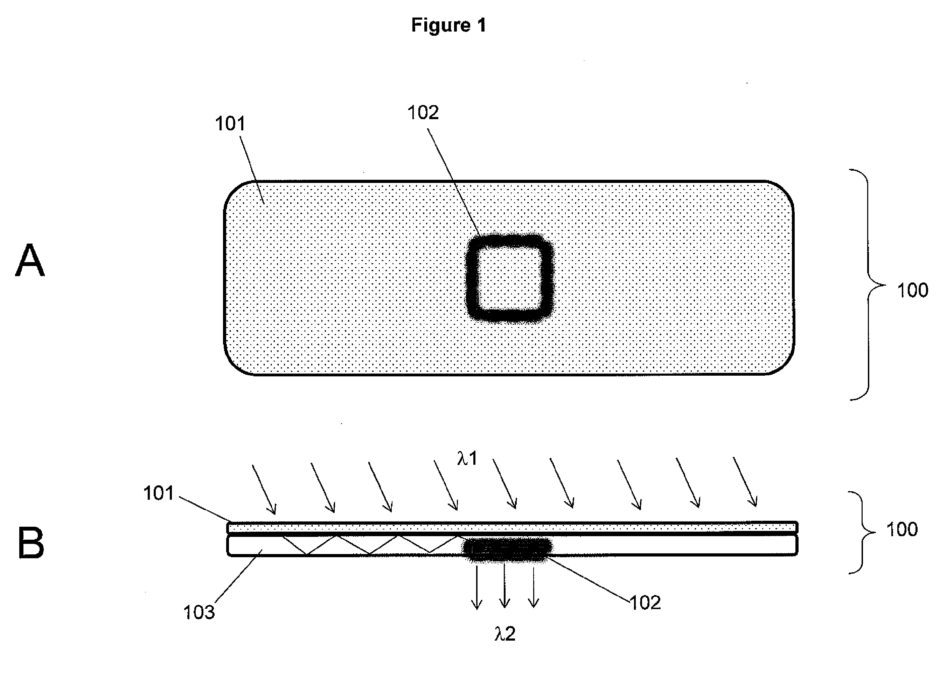

The top view and cross-section of a phototherapy plaster device of the present invention is shown in Figure 1A and 1B respectively, where 100 is the phototherapy device itself. A light collection surface 101 is capable of collecting ambient light and subsequently transmitting it to the light emission surface 102 through the lightguide 103. The lightguide can propagate the light via total internal reflection or through the use of mirror-like surfaces. Radiation λ1 collected by the plaster over large area can be converted to the output radiation λ2 having the desired spectrum and intensity for the treatment. The conversion may occur either in the radiation collection layer, the lightguide or at the radiation emitting surface. The conversion can be achieved, for example, by filtering light or converting it through the use of fluorescent dyes.

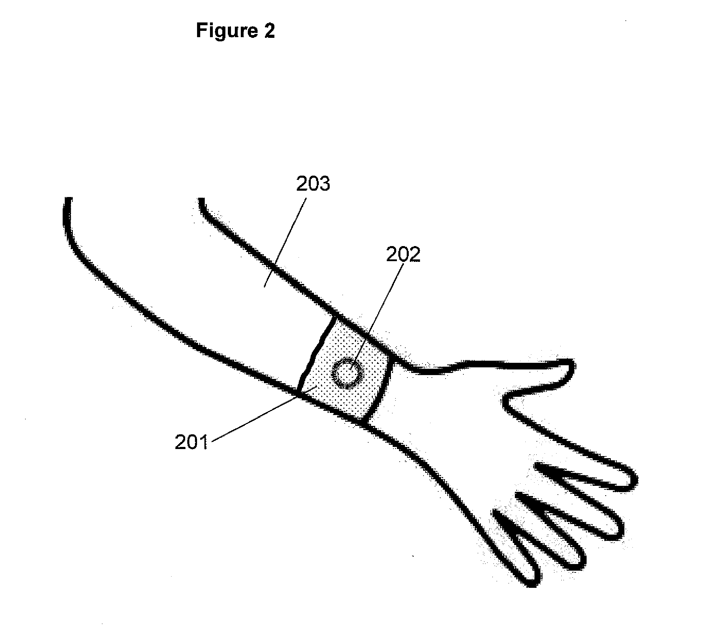

Figure 2 illustrates the use of a radiation treatment device of the current invention on an arm, for example in order to treat a wound. The light collection surface 201 may be shaped into a bandage in order to collect more light and deliver it to be treated through the radiation emitting surface 202.

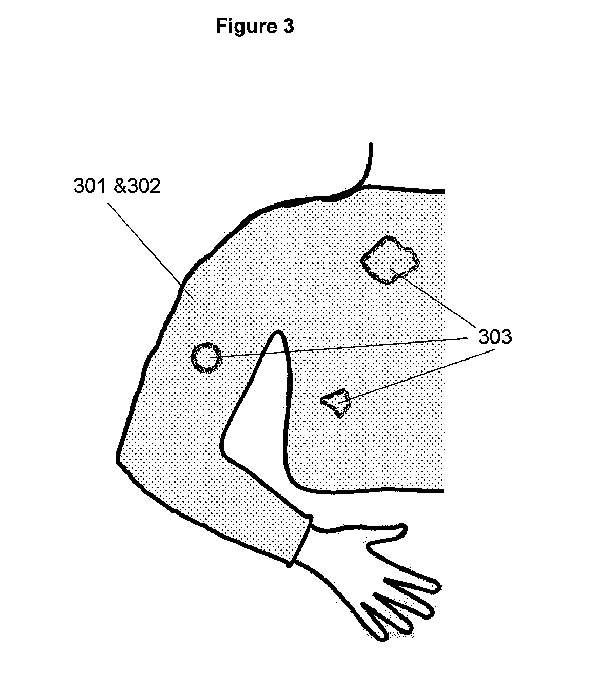

In another embodiment of the current invention, the radiation treatment device can be embedded into clothing as shown in Figure 3. The radiation collecting surface 301 and associated light guide 302 may comprise a significant portion or the entire area of the clothing. The collected light is then emitted through the emissive surface or surfaces 303 for localised delivery.

There are several important benefits with this approach. Since the phototheraphy device utilizes ambient light, it does not require a battery. A wearable lightweight device can be provided. The light collecting and lightguide layers can be made conformable and even flexible. Furthermore, it is possible to deliver light under clothing for treating a localised area. Many conditions such as acne, psoriasis and eczema respond well to slight elevation of light levels. The device can be made disposable, akin to a first aid wound plasters. The light accumulation surface is ideally 2-10000 times larger than the emissive area required for the localised treatment. More preferably, the light accumulation surface is 5-1000 times larger than the emissive area. For example, the emissive surface can be 20x5 cm while the emissive area can be 1x1cm. In turn the light output is increased to higher intensity at the emissive surface. The invention therefore enables the use of low intensity ambient light, yet provides sufficient light levels.

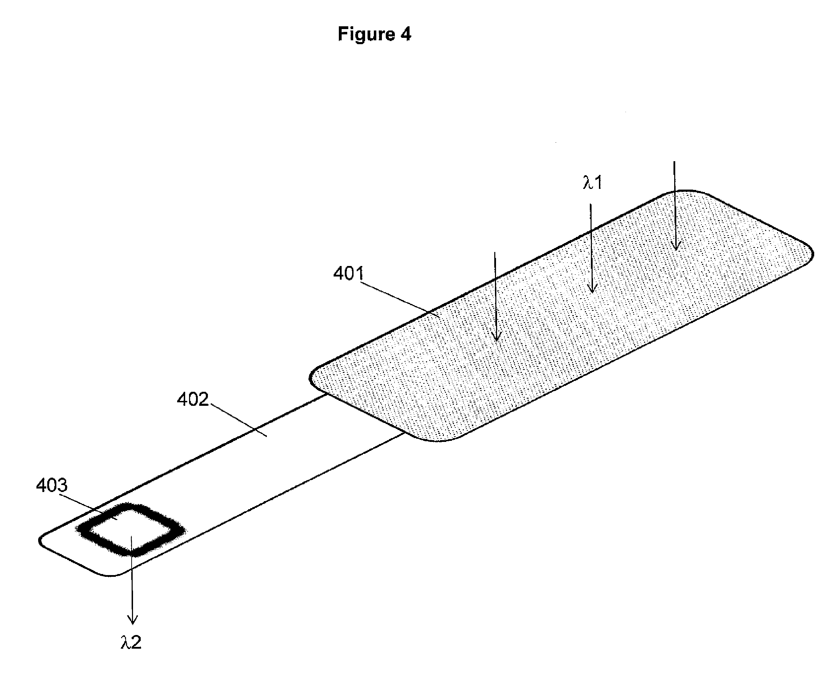

In another embodiment shown in Figure 4 the light collecting surface 401 and light emitting surface 403 can be offset while connected through a lightguide 402. Radiation λ1 absorbed by the plaster over large area of 401 can be converted and delivered to the output radiation λ2 having the desired spectrum and intensity for the treatment. This approach is useful to treat areas of the body that are not exposed to sufficient light or covered by clothing. Examples are underneath the forearm or even internal wounds required to stay open following operations. Surface of 401 can be attached to the outside of clothing, while 402 can take the shape of a flat or a fibre optic lightguide.

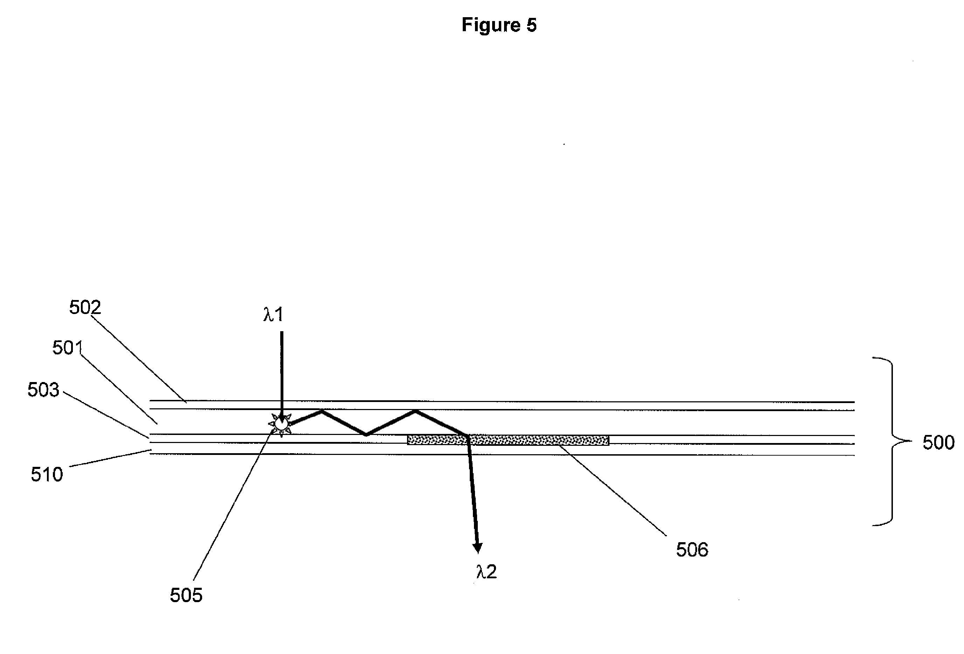

Figure 5 illustrates an example of cross-section of the inventive phototheraphy device 500. In this embodiment, the light collecting and lightguide surfaces comprise layers 501, 502 and 503. The lightguide core 501 is optionally doped with a luminescent dye 505. Layers 502 and 503 are low index materials designed to ensure total internal reflection of the light entering layer 501. The light propagates within the lightguide to the light outcoupling region 506 to provide light emitted for localised treatment. The light collection area of layer 501 has an area substantially larger than the treatment area 506. Light λ1 is absorbed by the dye 505 and is reemitted into layer 501 at wavelength λ2. The path of light is then guided via internal reflection to the eye region 506. The layer 503 may optionally be reflective, for example by metallisation. The area 506 comprises a light outcoupling feature such as embossed or moulded features. This region may also contain pigment particles to scatter light for efficient outcoupling. Any suitable outcoupling technique can be used. The device may optionally include a support 510 transparent to the therapeutic wavelengths of light at least in the area of 506. The support may optionally be coated with an adhesive at its perimeter for attaching it to the skin.

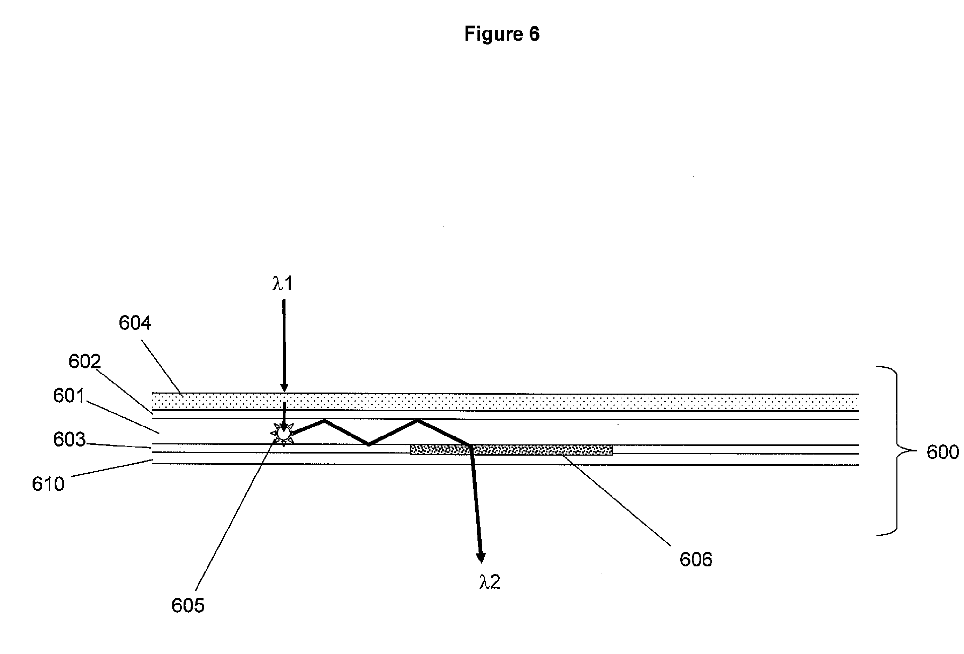

In an another embodiment shown in Figure 6 the phototheraphy device 600 comprises long decay phosphorescent materials in layer 604 for light collection. Phosphorescent layer 604 collects ambient light and subsequently re-emits it into the lightguide 601 adjacent to low index materials 602 and 603. The lightguide 601 is optionally doped with a luminescent dye 605. The light propagates within the lightguide to the light outcoupling region 606 to provide light emitted for localised treatment. The light collection area of layer 601 has an area substantially larger than the treatment area 606. Light λ1 absorbed by the device is stored by layer 604 and can be re-emitted over several hours. One or more phosphorescent and luminescent materials can be used in 604 and 601 in order to capture more ambient wavelengths of light and convert them to the desired output of λ2 at surface 606. The device may optionally include a support 610 transparent at least in the area of 606. The support may optionally be coated with an adhesive at its perimeter for attaching it to the skin. In this embodiment the invention enables very low intensity afterglow materials to be used, yet provides sufficient light levels for phototherapy treatment.

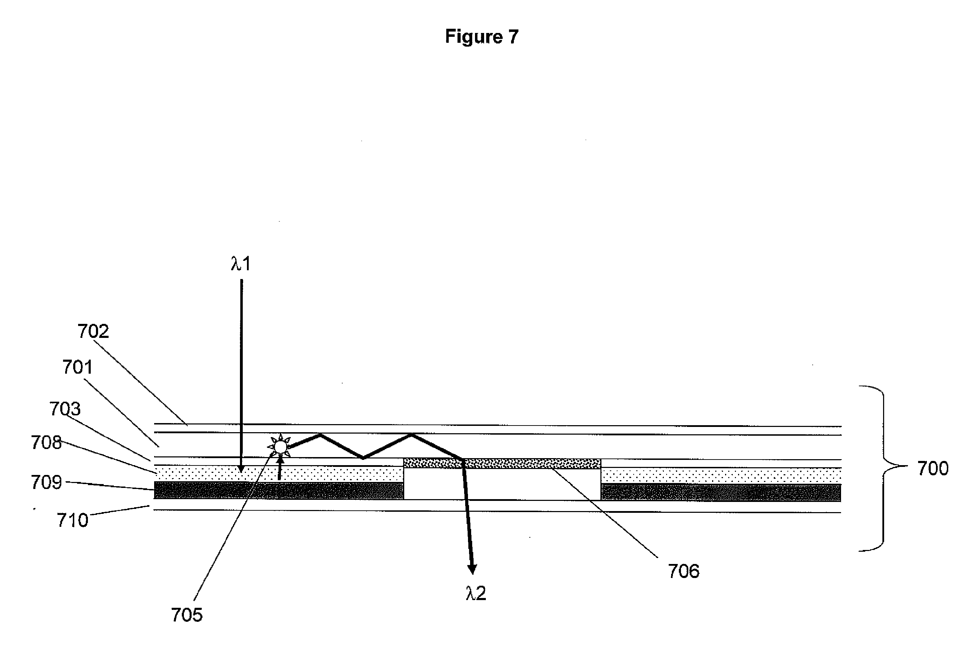

Figure 7 is a crossection view of a device 700 with a phosphorescent layer 708 disposed under the lightguide. The ambient light λ1 is absorbed by 708 and slowly re-emitted into the lightguide 701, disposed between low index layers 702 and 703. The lightguide 701 is optionally doped with a luminescent dye to couple efficiently light in. The guided light is then concentrated and emitted through outcoupling area 706 as λ2 for localised treatment. The device may include an optional reflective layer 709 to reflect light into lightguide 701. The device may further comprise a support 710.

The light guides described above employ adjacent layers of low optical index materials or reflective layers. These low index layers may also optionally comprise at least one luminescent dye to effectively couple light into the core of the lightguide. The dye may be used to define the spectra of the light for the treatment.

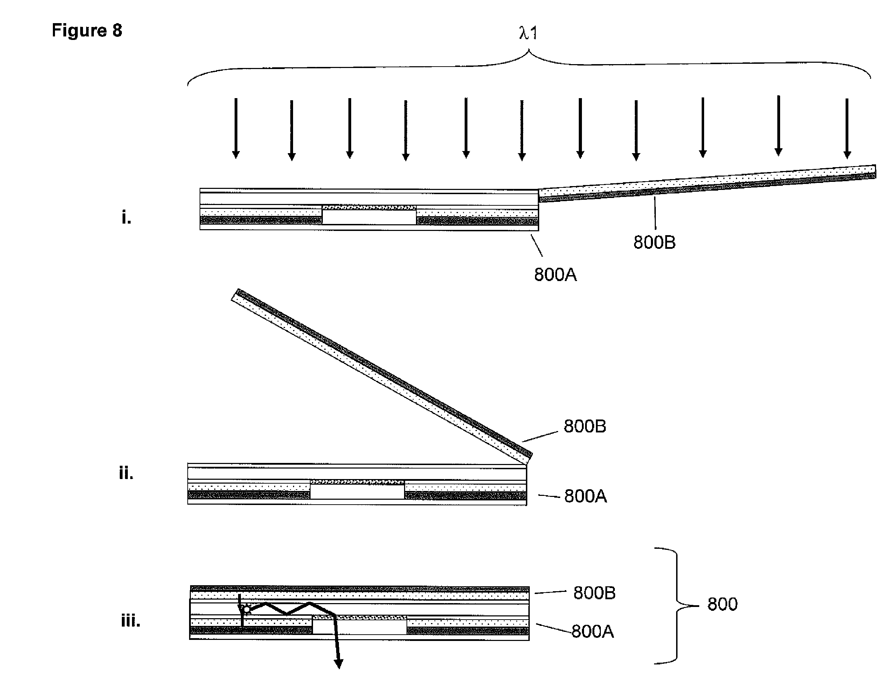

Figure 8 illustrates a phototherapy device 800 having a foldable structure with two sets of light collection surface segments 800A and 800B. In one embodiment, the surfaces comprise long afterglow phosphorescent layers in each segment. This device can be conveniently charged in the open state as shown in Fig 8(i). External light λ1 from the ambient excites the phosphorescent material causing it to glow following excitation. The device can be folded as shown in Fig. 8(ii). The device is shown in its closed state in Fig.8(iii). Light emitted from either side is captured in the lightguide and propagated toward the treatment area via internal reflection. In this embodiment the emissive area is significantly larger than the treatment area. For example, the total emissive area can be as large as 400 cm², while the area illuminating the tissue can be 1 cm².

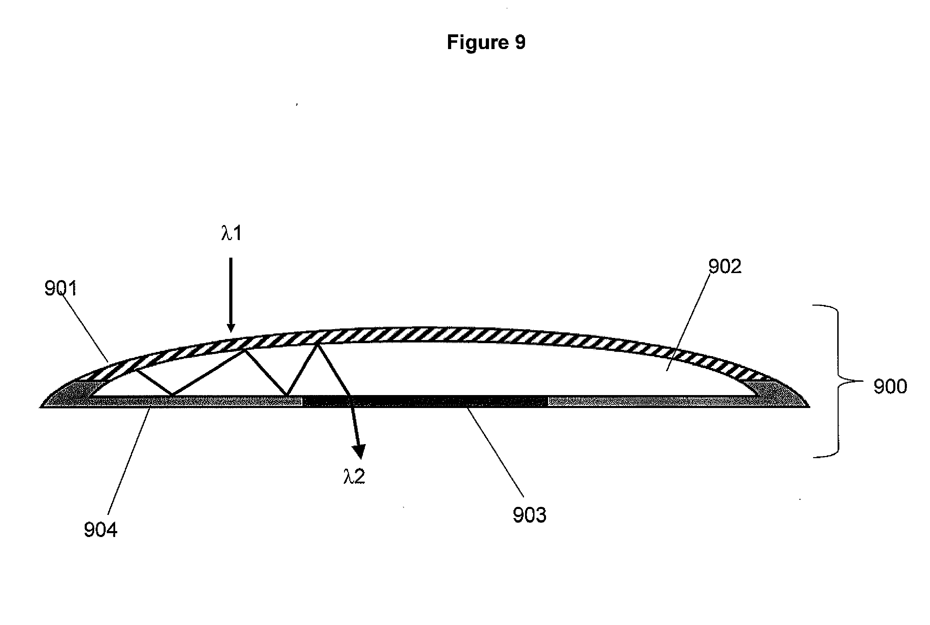

Figure 9 illustrates a preferred embodiment of a phototherapy device 900 in which the lightguide 902 is tapered or narrowed towards the edge of the device. This reduces the number of reflections for the light emitted at the edge due to the gradually increasing angle of reflection from the low index layers and/or reflective layers surrounding 902. The edge of the lightguide might also be rounded to reduce outcoupling and loss of light at the edge. In this embodiment light λ1 collected by light collection surface 901 is coupled into the lightguide material 902. The lightguide may optionally be doped with a dye. Outcoupling is effected via surface 903.

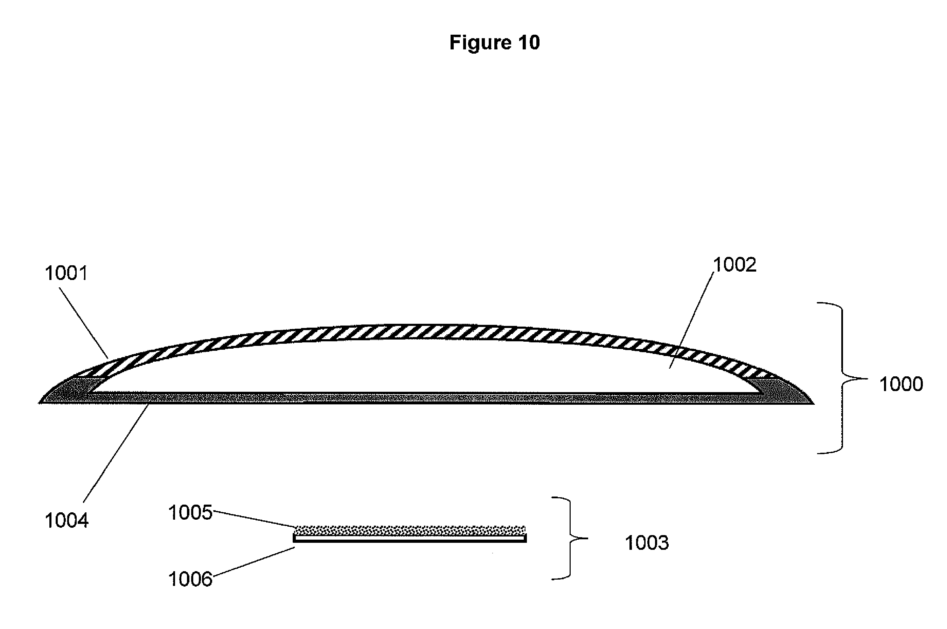

Figure 10 illustrates a preferred embodiment of a phototherapy device comprising of separate light collection section 1000 and a light outcoupling section 1003. In this embodiment 1000 does not have outcoupling features. Light is outcoupled by film 1003 when brought into contact by the light collection section 1000. This is achieved by providing a rough surface finish 1005 on 1003 that disrupts total internal reflection in 1000 when brought in contact with it. The surface finish may be provided by sharp moulded, embossed or coated features. For example 1005 can comprise a rough pigmented surface containing TiO₂ particles coated on the surface on support 1006. The support 1006 is transparent to allow light through. The light outcoupling film 1003 may be manufactured as a roll of tape and cut to shape and size desired for the treatment area.

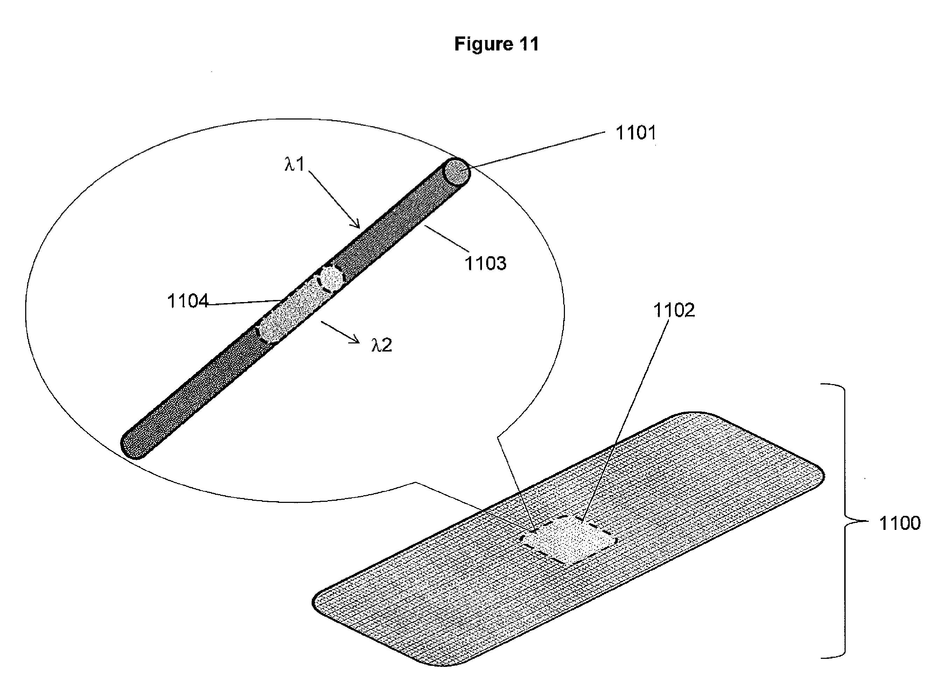

Figure 11 illustrates a preferred embodiment of a phototherapy device 1100 comprising several individual fibre lightguides 1101, each having a light collection surface 1103 and a light emitting surface 1104. In this embodiment light λ1 collected by light collection surface 1103 is coupled into the lightguide fibre 1101 and emitted through area 1104. The fibres are arranged in such a manner that outcoupling areas 1104 are located over section 1102 of the plaster. Each fibre collects light along its length and couples it out on a smaller section to deliver light over 1102 for localised radiation treatment. The fibres can be arranged in any suitable way, for example woven, in a grid or wound up randomly. The outcoupling regions can be defined post assembling the fibres, for example by embossing a sharp pattern over area 1102. In this embodiment, the treatment device can be made porous, or breathable. It can be appreciated that the outcoupling region 1104 can be formed on any portion of the fibres in a variety of ways. The outcoupling region for example can be comprised of the fibre ends. The internal layer structure of the lightguide fibres can be defined according to any layer structure described in Figures 5 and 6, the layers and coatings being concentric around the core of the lightguide.

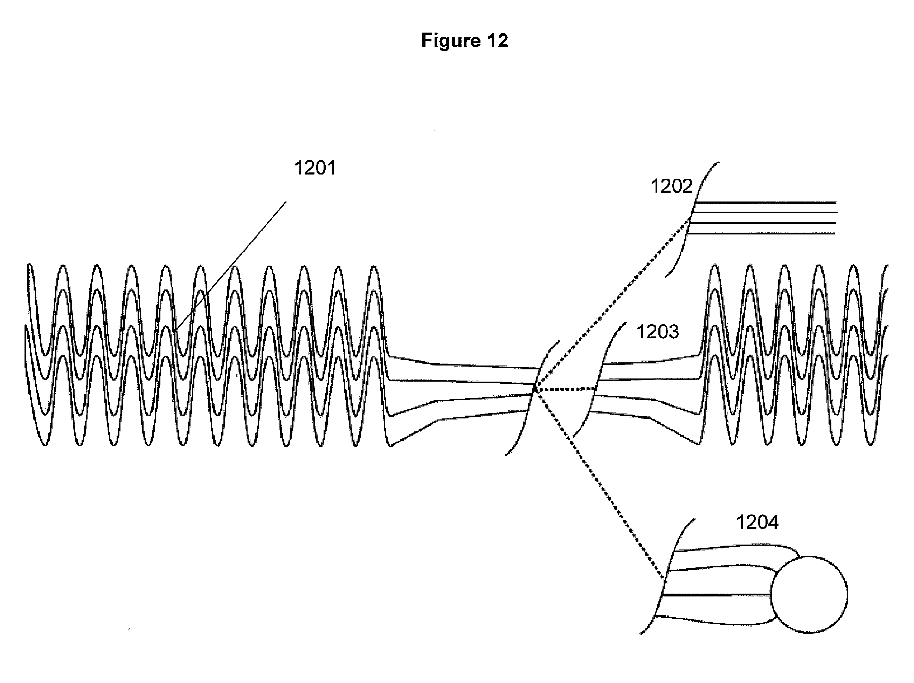

Figure 12 illustrates three variants for defining the emission area when fibre surfaces 1201 are used as the light collection area. The fibres in the emission area can be parallel as shown in 1202 or can be woven in a manner similar to the collection area as shown in 1203. Over these regions the fibre can be scored or treated so that the light can escape to the treatment area. The fibres may also be bundled into an outcoupling surface 1204 comprising scattering medium.

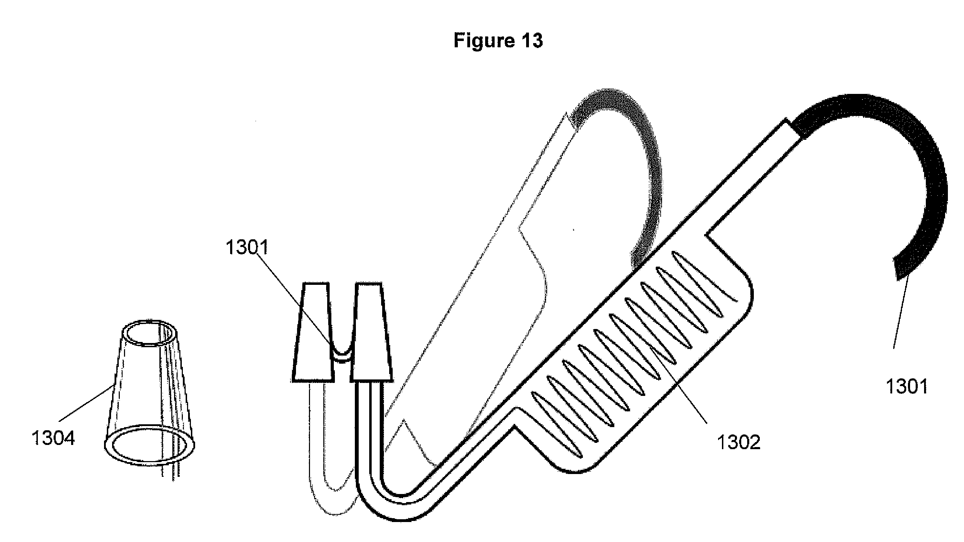

Figure 13 illustrates a device wearable on the face, with hooks 1301 to go over the ears holding the collection area 1302 and a clip to hold the emission area 1301 in the nose. The emission area of the fibres in this case is incorporated into a tube 1304, so that light may be directed into the nose and the wearer can also breathe through the nose.

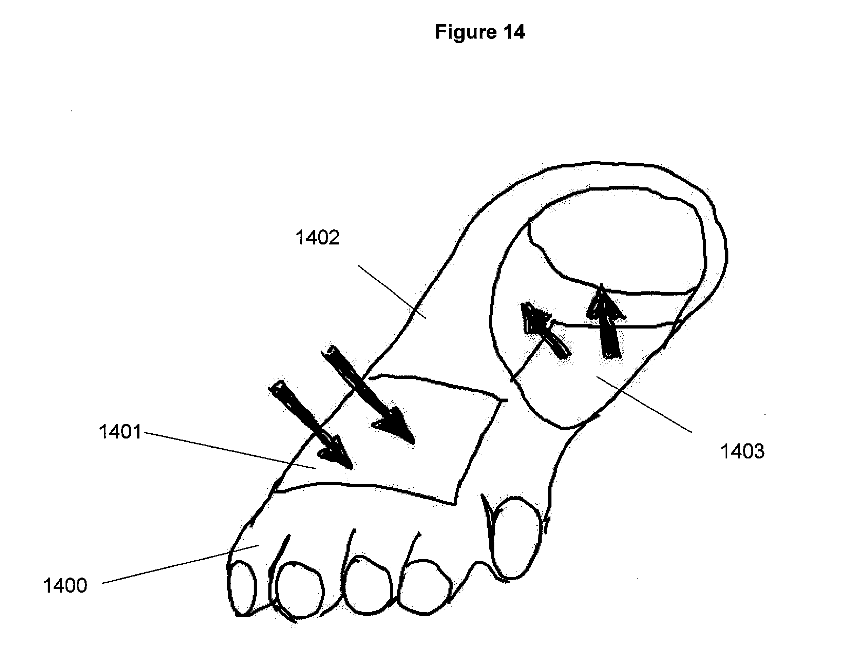

In another embodiment Figure 14 illustrates a wearable item, in this case a glove 1400, into which absorption surface 1401 is incorporated. The light is guided through the body of the glove 1402 and emitted at the inner surface 1403 shown here through the cut-through area. Analogously, the present invention can be incorporated into any clothing item such as shirts, shoes, hats and the like. Such devices are suitable for the treatment of conditions such as, but not limited to, cuts and grazes, joint sprains/swelling, Repetitive Strain Injury (RSI) and skin infections.

Any of the above embodiments may comprise at least one long afterglow phosphorescent layer in order to release light slowly. Such layers can be suitably charged by any external incident light such available in buildings, homes or offices. The device might also be charged by a dedicated light charger unit. The charger may be an enclosure containing one or more illuminating sources such as LEDs or incandescent bulbs. The charger may also be in the form of an illuminator sheet with which the phototherapy device is brought into contact.

In order to compensate for variations of light intensity, the phototheraphy device may comprise one or more photochromic layers. By including a photochromic member, it is possible to limit the initial brightness and make the output illumination more even with time. When the luminance of the ambient is lower, the photochromic layer becomes gradually more transparent, effectively providing a simple compensation mechanism.

The phototherapy device may also comprise a gel material to make soft contact with tissue. The gel may have additional functionality of cooling, for example, by placing it in a refrigerator prior to use. The gel may be part of the lightguide in the device.

### Light collection layers

In one embodiment the light collection surface comprises phosphorescent or luminescent materials that exhibit long glow after excitation. For example, following a light exposure they glow for minutes or hours. Such materials make it possible to provide battery free phototherapy devices with the capability to recharge the device for prolonged light emission. Materials with delayed phosphorescence excited by any electromagnetic radiation can be considered, for example ultraviolet light, visible light, infrared light or heat. Such materials can be formed into a film, sheet or coating to provide an light accumulation layer. The sheet or coating may comprise substantially the entire area of the device, coupling light into the lightguide described in the present invention.

The light collection layer should be structured in a manner that improves light coupling into the waveguide or on to the wavelength conversion materials. Such structuring may include the formation of 2D or 3D photonic crystals to direct the light emission or larger scale structures formed by, for example, deposition onto non-planar surfaces such as microprisms, or moulding into non-planar shapes.

Materials particularly relevant to the invention are long decay luminophore materials where the re-emitted light is slowly released over a period of time after initial excitation. Suitable materials include, but are not limited to, those described in US patent 5424006 and US Patent 5686022. Suitable materials include those manufactured by Nemoto & Company, Tokyo and are available under the brand name LUMINOVA having the general formula MAl₂O₄, where M is one or more metals selected from strontium (Sr), calcium (Ca), barium (Ba), magnesium ( Mg) activated by europium (Eu) and at least one co-activator selected from lanthanum (La), cerium (Ce), praseodymium (Pr), neodymium (Nd), samarium (Sm), gadolinium (Gd), dysprosium (Dy), holmium (Ho), erbium (Er), terbium (Tb), thulium (Tm), ytterbium (Yb), lutetium (Lu), tin (Sn), manganese (Mn) and bismuth (Bi). Chemical compositions of exemplary materials for use in the invention include, but are not limited to: SrAl₂O₄:Eu; SrAl₂O₄:Eu, Dy; SrAl₂O₄:Eu, Nd; Sr₄Al₁₄O₂₅:Eu; Sr_{0.5} Ca_{0.5} Al₂O₄: Eu , Dy; BaAl₂O₄: Eu, Nd; BaAl₂O₄:Eu, Sn; ZnS:Cu; ZnS:Cu, Co; ZnS:Mn; ZnS:Ag; BaMgAl₁₀O₁₇:Eu; BaMgAl₁₀O₁₇:Mn, Eu; Sr₂P₂O₇:Eu; CaWO₄; CaWO₄:Pb; SrGa₂S₄:Eu; CePO₄:Tb; MgWO4; Y₂O₃:Eu; Y₃Al₅O₁₂:Ce; (Ba₁₋ₓSrₓ)5(PO₄)₃(F,Cl):Eu; (Y_{1-x-y}GdₓLu_{y})₃(Al_{1-y}Ga_{y})₅O₁₂:Ce; (Y₁₋ₓGdₓ)₂O₃:Bi,Eu; (Y₁₋ₓPₓ)₂O₄:Eu; YVO₄:Eu; Y₂O₂S:Eu.

### Lightguides:

The lightguide may comprise substantially the whole area of the phototheraphy device. Preferably the lightguide comprises between 5% to 100% of the device area. More preferably the lightguide comprises between 10% to 95% of the device area. The lightguide is the same size or larger than the light collection surface. Suitable lightguide constructions include a polymer core with a high index material and surrounding low index polymer layer(s), which may extend to both sides of the lightguide. Where required for outcoupling, an additional high index coupling layer such as narrow angle high gain GRIN diffusers (for example those offered by Microsharp Ltd) can be placed on the lightguide. Where a wavelength conversion layer is used, an appropriate position would be between the low index cladding and high index core, in order to take advantage of Supercritical Angle Fluorescence (SAF) as described by Enderlein et al (Highly Efficient Optical Detection of Surface-Generated Fluorescence. Appl. Opt. 38 (4) 724-32 (1999) which allows the preferential injection of converted light into waveguided modes of the high index core. Appropriate materials for the waveguide should have a high degree of transparency in the spectral region of interest, and where required should be flexible and appropriate for the particular method of manufacture. Low index materials therefore may include MY-132 (MY Polymers Ltd), with refractive index 1.32. A high index core could include material such as polysiloxanes (WO/2003/011944) with refractive index of up to 1.581. This combination of materials would have a critical angle of 58 degrees, and SAF emission from the interface would couple up to approximately 70% of light directly into the waveguided modes.

Other lightguide materials include polystyrenes, poly(ethylene terephthalate), a transparent polyolefin, in particular a clarified polyolefin, for example clarified polypropylene, Poly(methyl methacrylate) (PMMA), transparent polyamide or polycarbonate and perfluorinated polymers such as polyperfluorobutenylvinylether, polysulphones,polyether sulphones and polyacrylates. PMMA and polycarbonate are two transparent thermoplastics of choice because of their ease of processing, their availability on the market, their high transparency in the visible range and refractive index in the visible range. Other materials which are recommended for use with fluorescent dyes include styrene-butadiene. For the low index cladding, polymers such as TEFLON FEP, PCTFE (polychlorotrifluoro-ethylene) or PVDF as well as UV curable low index polymers such as the OPTI CLAD (manufactured by Ovation Polymers) series, may be chosen, which have refractive indices as low as 1.33. Further examples of polymers can be found in "Encyclopedia of Polymer Science and Engineering", 2nd Edition, J Wiley and Sons and "Polymer Handbook", 4th ed.; John Wiley & Sons, 1999.The lightguides may be further surrounded by reflectors, as described in a later section in order to recycle non-waveguided light, both allowing light from the initial light wavelength to be downconverted on later passes and also recycle non-SAF emitted light. In general, structures constructed so that the majority of the light is waveguided by Total Internal Reflection (TIR) will have better device performance. Additionally the high index core of the lightguide may be doped with light accumulation or wavelength converting chemical. The ends of the lightguides may be squared and mirrored, or they may be tapered to a point or curved edge such as a parabola such that waveguided light may be turned and reflected towards the centre of the guide again with minimal reflections from metallic surfaces. The lightguide may also be a gel material within a polymer shell.

### Wavelength conversion materials

The lightguide or light collection surface may comprise wavelength conversion materials such as dyes. Such materials can aid coupling of light into the lightguide. Wavelength conversion is also an approach to adjust the wavelength emitted for the treatment. By using such materials it is possible to convert less effective wavelength light into an effective spectrum. Suitable materials or mixtures of materials are those which absorb light at a first wavelength and subsequently emit light at a second wavelength which is different from the first wavelength. The emitted light might be result of fluorescence and/or phosphorescence depending on the type of material or mixture of materials. In addition the nature of the material or mixture of materials and associated energy conversion mechanisms will dictate if the wavelength emitted is larger or smaller than that of the absorbed light. Light can be absorbed by one species and re-emitted by a second species following non-radiative energy transfer.

Suitable wavelength conversion materials may be of any type including but not limited to coumarins; perylenes; xanthenes; thioxanthenes; fluoresceins; rhodamines; azlactones; methines; oxazines; thiazines; phtalocyanines; stilbenes; distilbenes; distyrenes; azomethines; phenanthrenes; rubrene; quinacridones; naphtalimides; methines; pyrazolones; quinophthalones; perinones; anthraquinones. Materials particularly relevant to the invention are those which can be efficiently doped into light guide materials without compromising their optical function and which exhibit a high quantum yield. Of particular interest are fluorescent dyes manufactured by BASF and available under the brand name LUMOGEN F Dyes. Other commercial materials of interest include those available under the following brand names MACROLEX (Bayer), HOSTASOL (Clariant), THERMOPLAST (BASF), SOLVAPERM (Clariant), SANDOPLAST (Clariant), AMAPLAST (Color-Chem).

Quantum dot materials may also be used to adjust the emission characteristics of the device. Suitable quantum dots materials include but are not limited to CdSe, CdTe, ZnS, ZnTe, ZnSe, CdS, HgS, HgSe, HgTe, CdTeSe, CdTeS, ZnSSe, ZnTeSe, ZnSTe, CdZnSe, CdZnTe, CdZnS, GaAs, GaP, GaSb, GaN, InN, InP, InAs, InSb, InGaP, InGaAs, InGaN, AlInGaN, AlInGaP, AlInGaAs, Si, Ge.

The above wavelength conversion materials may also be used as part of the light collection surface or the outcoupling surface.

### Outcoupling methods

The phototherapy device comprises at least one outcoupling region as an exit window to shine light onto the target area. Outcoupling from this region may be achieved through the use of scattering, embedded nanoparticles and/or quantum dots which may be doped or undoped, of various shapes and orientations such as rods, triangles or spheres, optical interference structures such as diffraction gratings or holographic structures, photonic crystals which may be either 2D or 3D, macroscopic shaped structures such as, but not limited to, micropyramids and microprisms, microlenses, lens arrays, GRIN structures, fiber optics for either scattering or redirection of light through waveguiding or other coupling methods through variation of refractive index. The outcoupling region may either be directly on the side of the escape window area forming part of the window, it may be an external component attached to the window, or it may be on the opposite side of the waveguide to the window (i.e. diffractive components) Additionally bulk components such as scattering particles, nanoparticles and lumophores may be present in the waveguide near the window for outcoupling, or they may form part of external components. Concave or convex outcoupling schemes may be employed. Outcoupling materials may either be integral parts of the device itself, or may also consist of index matching gels or oils such as mineral oil (often marketed as baby oil) which may be applied directly to the skin or treatment area, and then the escape window of the device positioned above them.

### Reflectors

Reflectors will have two primary purposes. The first is to ensure that light that is not wavelength converted and will be substantially reflected through the wavelength conversion region so that a significant proportion of light will be converted on later passes as opposed to being absorbed and lost. The second is to provide supplementary waveguiding to light intended for emission that has not been injected into supercritical waveguided modes in the waveguide core. In the case of the SAF layer above, this would make up around 30% of the light. The reflectors may consist of periodic or aperiodic dielectric multilayers which may be either isotropic or anisotropic in nature, with the particular arrangements of refractive indices, thicknesses and index ellipsoid orientations chosen to optimise reflections of light which is not captured within the waveguide to either ensure its passage to the outcoupling region or to increase the chances of absorption and re-emission at the wavelength conversion layers. Such materials may either be custom arrangements of layers which may be extruded or laminated onto the core or commercially available films. The choice of materials may also be chosen to allow their dual use as barrier layers to lengthen the lifetime of the devices. In the event that no practical arrangements can be found, it would also be possible to used metallic or metallised layers as a reflector. However for large numbers of reflections, the efficiencies of metallic layers are significantly poorer.

### Optical modulation layer

The device may optionally comprise one or more optically variable layers for modulation of the light coupling. This may for example be a photochromic light absorber (such as silver chloride) or may change refractive index such that index matching between device layers increases or decreases according to the desired conditions. For example, an optical modulation layer can be inserted on either side of the light collection surface. Alternatively, the optical modulation layer may be inserted on either side of the light emission surface or even within the lightguide. Such layers that change properties based on conditions including but not limited to, pH and chemicals (chemochromic), humidity (hydrochromic), temperature (thermochromic) and pressure may also be used. The layer may consist of pure photochromic material or a doped material such as polymer. The range of suitable materials is large and includes inorganic materials such as zinc halides and silver chloride, as well as organic materials such as spirooxazines, which have fast switching in gels and spiropyrans, which can be cross linked with other molecules. Such organic molecules as these may be tuned to specific wavelength switching requirements.

### Shape, form of implementation

The phototheraphy device is ideally flexible or conformal. The device may be provided as a plaster or bandage. The form factor and bend angles of the waveguide should remain small with respect to the thickness of the waveguide in order to ensure an acceptable level of total internal reflection is maintained. The lightguide may comprise a soft material such as a gel. The gel may provide an additional cooling function to soothe the treatment area. The external surface of the device may be covered with a fabric material or other soft layer or layers such as a foam. These layers can have the function to provide vapour transmission.

The phototherapy device can also comprise a photochemical and/or a photo-pharmaceutical agent in the form of a gel, ointment or cream. Such agent can exist as a layer on the phototherapy device which will couple to the skin as the device is applied, can be delivered to the skin independently by direct application or via the use of a impregnated material. This material may also provide a dual function of improving light coupling to the target tissue though index matching.

### Method for manufacturing

The radiation treatment device can be manufactured by a variety of techniques. The lightguide is preferably manufactured by moulding, lamination, stretching, forming of the lightguide as well as emissive components. The outcoupling surface and features can be formed in a single moulding step when forming the device or defined in a second step by embossing or coating further layers. The light collection surface may be laminated or attached to the lightguide using an adhesive. A light collection material may also be directly coated onto the lightguide by solution coating techniques such as screen-, flexo-, gravure-, or inkjet printing, spray coating or dip coating. Light accumulation materials may also be deposited on the lightguide by evaporation.

### Examples

### Example 1: passive phototherapy plaster for treatment of acne

Acne is a condition affecting a large percentage of the population. Illumination with red and blue light offers an alternative to drug based treatments of such condition (Papageorgiou et al.; Br. J. Dermatol.; vol. 142; p. 973; 2000).

A number of portable commercial phototherapy devices are available for the specific treatment of acne such as Dermastile by Lumiport and Clear by Lumie . However these devices are not particularly convenient to the user as they require to be held in position.

Recently a PDT approach also suggests that combining illumination with a photo-pharmaceutical further improves the effectiveness of the treatment (Taylor et al.; Br. J. Dermatol.; vol. 160; p. 1140; 2009 and Riddle et al.; J. Drugs Dermatol.; vol. 8; p. 1010; 2009).
This example describes a plaster like passive phototherapy device which could be used to provide the necessary therapeutic illumination for simple phototherapy or photo-dynamic therapy for the treatment of acne. The device is wearable and poses minimum constraints to the user.
A circular light guide less than 2mm in thickness and 10mm in diameter comprises a light collection area with diameter approximately equal the device diameter and a circular light delivery area, 3 mm in diameter, situated on the face opposite to the light collection area with the low index lightguide cladding removed from this delivery area. The light guide is tapered at its edge as shown in figure 9 to intensify the transport of light towards the central part of the device. The adhesive layer raises the lightguide above the skin leaving a small cavity. This cavity then may be either pre-filled with a gel, or a gel may be placed on the skin and the plaster placed over it. The gel would have a refractive index equal to or higher than the lightguide core allowing coupling from the lightguide directly to the target area. Alternatively this cavity could be filled with a soft silicone rubber to serve the same purpose.

The material is formed by injection moulding silicone resin containing 0.1% Lumogen F Red 305 (BASF) fluorescent dye. The light guide is coated on both sides with a 5 microns thick lower index polymer such as polychlorotrifluoro-ethylene. On the light collection side the device is then coated with a layer containing luminophore such as Luminova B-300 (Nemoto). In order to improve light storage a dichroic filter film can be optionally formed other the luminophore so that it is transparent to light with wavelength being absorbed by the luminophore and reflects light emitted as a result.

The ambient radiation collected by the device excites the luminophore which then re-emits light with peak wavelength around 450 nm. A large proportion of that light will enter the light guide where it will be absorbed by the fluorescent dye and re-emitted as light with peak wavelength around 620 nm. As a result the light exiting the device at the light delivery area will be a mixture of red and blue light.

While delivering therapeutic light to the target area under ambient light illumination the device will also provide some degree of illumination after the source of ambient radiation has been removed, for example when the user goes to sleep.

### Example 2: passive phototherapy eye mask for treatment of rethinopathy

In patent application GB2410903A, Arden describes an eye mask device using inorganic LED light sources to illuminate the eyes of patients suffering from retinal diseases during their sleep. Because the treatment is carried out during sleep a phototherapy device with minimum discomfort for the user is advantageous. Because of the low light intensity required and the long illumination time available a passive phototherapy device using luminophore for slow release of light is particularly suitable. This example describes such a device.

A light guide sheet element (less than 2 mm in thickness) could be moulded in the shape of an eye mask as to cover the eye of a patient. The element is formed by injection moulding silicone resin containing 0.1% of Lumogen F Green 850 (BASF). The light guide is coated on both sides with a 5 microns thick lower index polymer such as polychlorotrifluoro-ethylene.

The light collection is defined by an area approximately equivalent to that of the said light guide element facing away from the patient's face. It is coated with a layer containing luminophore such as Luminova BGL-300 (Nemoto). In order to improve light storage a dichroic filter film can be optionally formed other the luminophore so that it is transparent to light with wavelength being absorbed by the luminophore and reflects light emitted as a result.

On the opposite side, two light delivery areas are defined to coincide with the eye of the patient so that the light emitted by the phototherapy device can illuminate the eyelids of the patient. These areas would assist outcoupling in one of a number of ways from surface structuring to the attachment of a soft sac containing mineral oil or silicone gel of an equal or higher refractive index to the core. This would allow light to be coupled directly from the lightguide core into a non-waveguiding structure directly over the eye, and allowing light into the eye. The use of a gel further assists comfort and coupling between the lightguide mask and eye.

The ambient radiation collected by the device excites the luminophore which re-emits light with peak wavelength around 490 nm. A large proportion of that light will enter the light guide where it will is absorbed by the fluorescent dye and re-emitted as light with peak wavelength around 500 nm which is adequate for absorption rod cells.
A variation of the same device would be the use of a foldable design as described in figure 8 to increase the collection area.

### Example 3: passive phototherapy canula for hay fever relief

Allergic rhinitis, or hay fever, is a common condition associated with the inflammation of the nasal airways brought about by exposure to allergens such as pollen or dust. Drugs available to relieve such condition are often associated with side effects. Intranasal phototherapy has been found to be an effective way of helping with such condition (Neuman at al; Ann. Allergy Asthma Immunol.; vol. 78; p. 399; 1997).

Commercial devices are available to deliver suitable wavelength of light into the nose of a patient such as Haylight by Lumie. The devices rely on the use of red and near-IR emitting inorganic LEDs and are not particularly convenient as their use prohibits air movement through the nose and the nasal light probes need to be connected to a power source. In addition due to the nature of the devices replacement probes are often required increasing the cost of the treatment and its environmental impact.

This example describes a lightweight passive phototherapy device which can deliver suitable wavelength of light into the nose of a patient without the need for any external wires. Such device is also easily disposable.

As described in figure 13, fibre shaped light guide elements can be used to collect ambient light and deliver light with suitable wavelength into the nasal cavity. In order to maximise light collection the fibres can be arranged in a way that maximises their surface area per physical area of the light collection potion of the device. For delivery the fibres can be bundled into a tube allowing easy insertion into the nasal cavity while minimising obstruction.

In order to deliver both visible red and near-IR radiation two types of fibres are used. The first type is doped with a fluorescent or phosphorescent material absorbing ambient light and emitting in the red, for example Lumogen F Red 305 (BASF). The cladding of the fibre can be removed where they are inserted into the light delivery tube to allow for the light to be emitted towards the target area.

The second type of fibre type is doped with a fluorescent or phosphorescent materials absorbing ambient light and emitting in a wavelength region which is absorbed by a near-IR emitting fluorescent or phosphorescent material. The latter is present in a layer that can be coated on the light delivery end of the fibre after cladding has been removed. The near-IR emitting phosphorescent material can be FL-940 (Moltech GmbH) used in combination with the fluorescent dye Lumogen F Blue 650 (BASF) which would be embedded in a higher index layer such as PMMA, which then coats the stripped fiber ends allowing transmission from the fiber into the new high index layer containing the near IR phosphor. The end of the fibres situated in the light collection area of the device can be metallised to reduce light leakage or alternatively can be looped back so that both ends of the fibre terminate in the nasal cavity.

It will be appreciated by persons skilled in the art that the above embodiments have been described by way of example only and not in any limitative sense, and that various alterations and modifications are possible without departure from the scope of the invention as defined by the appended claims.

## Claims

1. A wearable apparatus for directing electromagnetic radiation onto at least one tissue area, the apparatus comprising:
at least one radiation collection surface for receiving incident electromagnetic radiation;
at least one radiation emitting surface adapted to direct electromagnetic radiation onto at least one target area of tissue to be treated;
radiation guide means adapted to receive electromagnetic radiation received by at least one said radiation collection surface and to direct at least part of said electromagnetic radiation to at least one said radiation emitting surface, wherein a total surface area of the or
each said radiation collection surface is larger than a total surface area of the or each said radiation emitting surface; and
coupling means for increasing the proportion of electromagnetic radiation received by at least one said radiation collection surface which enters said radiation guide means.

2. An apparatus according to claim 1, wherein the apparatus is adapted to capture ambient light, direct at least part of said light onto a target area of tissue to be treated, and to adjust spectral properties and/or intensity of at least part of said light.

3. An apparatus according to Claim 1 or 2, wherein at least one said radiation emitting surface is provided on at least one detachable member.

4. An apparatus according to any one of the preceding Claims, wherein the radiation guide means comprises a radiation transmitting portion including at least one first layer having a respective first refractive index and at least one second layer having a respective second refractive index, wherein said first refractive index of at least one said first layer is higher than the respective second refractive index of at least one second layer in contact with said first layer.

5. An apparatus according to any one of the preceding claims, further comprising spectral adjustment means for adjusting spectral characteristics of electromagnetic radiation delivered to the target treatment area.

6. An apparatus according to claim 5, wherein said spectral adjustment means includes at least one material having a transmissivity to radiation varying with radiation intensity.

7. An apparatus according to any one of the preceding claims, wherein said coupling means includes at least one phosphorescent or luminescent material provided at at least one said radiation collection surface and/or said radiation guide means and/or at least one said radiation emitting surface.

8. An apparatus according to any one of the preceding claims, wherein the radiation guide means further comprises at least one reflective portion.

9. An apparatus according to any one of the preceding claims, wherein the radiation guide means comprises at least one radiation transmitting portion having tapered thickness adjacent at least one edge portion thereof.

10. An apparatus according to any one of the preceding claims, wherein the radiation guide means comprises a radiation transmitting portion, wherein the radiation transmitting portion is rounded adjacent at least one edge portion thereof.

11. An apparatus according to any one of the preceding claims, wherein at least one said radiation emitting surface is adapted to conform to a shape of the tissue to be treated.

12. An apparatus according to any one of the preceding claims, wherein at least one said radiation emitting surface includes at least one coolable material.

13. An apparatus according to any one of the preceding claims, wherein at least one said radiation emitting surface is adapted to be moved between a respective first position, in which said surface is adapted to direct radiation into said radiation guide means, and a respective second position, in which said surface is adapted to be exposed to exciting radiation.

14. An apparatus according to any one of the preceding claims, wherein the apparatus is integrated into clothing.

15. An apparatus according to any one of the preceding claims, further comprising at least one photochemical and/or photo-pharmaceutical agent.
